(19) **Europäisches Patentamt**
**European Patent Office**
**Office européen des brevets**

(11) **EP 2 237 878 B1**

(12) **FASCICULE DE BREVET EUROPEEN**

(45) Date de publication et mention de la délivrance du brevet:
**15.07.2020   Bulletin 2020/29**

(21) Numéro de dépôt: **08865288.8**

(22) Date de dépôt: **16.12.2008**

(51) Int Cl.:
*C07C 29/76* (2006.01)          *B01J 20/18* (2006.01)
*B01J 20/28* (2006.01)          *B01J 20/32* (2006.01)
*C07C 7/13* (2006.01)          *C07C 15/08* (2006.01)
*C07C 37/82* (2006.01)          *C07C 201/16* (2006.01)

(86) Numéro de dépôt international:
**PCT/FR2008/052316**

(87) Numéro de publication internationale:
**WO 2009/081023 (02.07.2009 Gazette 2009/27)**

(54) **ADSORBANTS ZEOLITIQUES AGGLOMERES, LEUR PROCEDE DE PREPARATION ET LEURS UTILISATIONS**

AGGLOMERIERTE ZEOLITHISCHE ABSORPTIONSMITTEL, HERSTELLUNGSVERFAHREN DAFÜR UND VERWENDUNG DAVON

AGGLOMERATED ZEOLITIC ABSORBENTS, METHOD OF PREPARING THEM AND USES THEREOF

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité:  **20.12.2007   FR 0760088**

(43) Date de publication de la demande:
**13.10.2010   Bulletin 2010/41**

(73) Titulaires:
• **ARKEMA FRANCE**
  **92700 Colombes (FR)**
• **IFP Energies nouvelles**
  **92500 Rueil-Malmaison (FR)**

(72) Inventeurs:
• **BOUVIER, Ludivine**
  **64140 Billere (FR)**

• **KIEGER, Stéphane**
  **78500 Sartrouville (FR)**
• **LAROCHE, Catherine**
  **69390 Vernaison (FR)**
• **LEFLAIVE, Philibert**
  **69780 Mions (FR)**
• **FRISING, Tom**
  **92000 Nanterre (FR)**

(74) Mandataire: **Bandpay & Greuter**
  **30, rue Notre-Dame des Victoires**
  **75002 Paris (FR)**

(56) Documents cités:
**WO-A-99/10096          WO-A-2008/009845**
**FR-A- 2 863 909          FR-A- 2 903 978**

**Description**

L'invention concerne des adsorbants zéolitiques agglomérés à base de poudre de zéolite de type faujasite X à faible teneur en silice et à petits cristaux, échangée au baryum ou à base de zéolite de type faujasite X à faible teneur en silice et à petits cristaux, échangée au baryum et au potassium.

Ces adsorbants peuvent être utilisés plus particulièrement pour la production de paraxylène très pur à partir d'une charge d'hydrocarbures aromatiques contenant des isomères à 8 atomes de carbone.

L'art antérieur a reconnu que les adsorbants constitués de zéolites X ou Y échangées au moyen d'ions tels que le baryum, le potassium ou le strontium, seuls ou en mélange, sont efficaces pour adsorber sélectivement le paraxylène dans un mélange d'hydrocarbures aromatiques.

Les brevets US 3 558 730, US 3 558 732, US 3 626 020 et US 3 663 638 montrent que des adsorbants comprenant des aluminosilicates échangés par du baryum et du potassium ou par du baryum seul (US 3 960 774) séparent efficacement le paraxylène d'un mélange d'isomères aromatiques à 8 atomes de carbone ($C_8$).

Un mode de préparation de ces adsorbants est par exemple décrit dans US 3 878 127 et consiste à traiter, dans la soude à chaud, des agglomérés (zéolite X + liant) de rapport $Na_2O/Al_2O_3$ strictement inférieur à 0,7 afin de remplacer les cations échangeables de la zéolite (tels que protons ou cations du Groupe IIA), par du sodium préalablement à un échange baryum ou baryum + potassium, l'échange préalable au sodium permettant à une plus grande quantité d'ions baryum ou baryum + potassium d'être ajoutés à la structure zéolitique.

Ces adsorbants sont utilisés comme agents d'adsorption dans les procédés en phase liquide, de préférence de type contre-courant simulé, similaires à ceux décrits dans US 2 985 589, qui s'appliquent entre autres aux coupes de $C_8$ aromatiques.

Les zéolites qu'on rencontre dans l'art antérieur pour la séparation des xylènes appartiennent au type structural de la faujasite, tout d'abord décrites dans US 2 882 244 et US 3 130 007, qui sont des silico-aluminates cristallisés possédant des cages de taille parfaitement déterminée connectées dans les trois dimensions.

US 6 884 918 préconise une faujasite X de rapport atomique Si/Al entre 1,15 et 1,5. US 6 410 815 enseigne que des adsorbants zéolitiques tels que décrits dans l'art antérieur mais pour lesquels la faujasite est à faible teneur en silice et présente un rapport atomique Si/Al proche de 1 (que l'on appellera LSX, abréviation de Low Silica X dont la traduction française est zéolite X à faible teneur en silice) sont avantageusement utilisés pour la séparation du paraxylène.

Dans les références listées ci-dessus, les adsorbants zéolitiques se présentent sous forme de poudre ou sous forme d'agglomérés constitués majoritairement de zéolite et jusqu'à 15 à 20% en poids de liant d'agglomération inerte.

La synthèse des zéolites LSX s'effectuant le plus souvent par nucléation et cristallisation de gels de silico-aluminates, on obtient des poudres dont l'emploi à l'échelle industrielle est particulièrement malaisé (pertes de charges importantes lors des manipulations) et on préfère les formes agglomérées, par exemple sous forme de granulés ou de grains, qui ne présentent pas les inconvénients inhérents aux matières pulvérulentes.

Ces agglomérés, qu'ils soient sous forme de plaquettes, de billes ou d'extrudés, sont en général constitués d'une poudre de zéolite, qui constitue l'élément actif (au sens de l'adsorption) et d'un liant destiné à assurer la cohésion des cristaux sous forme d'agglomérés et à conférer à ces derniers une résistance mécanique suffisante pour résister aux vibrations et aux mouvements auxquels ils sont soumis au cours de leurs mises en oeuvre.

La préparation de ces agglomérés s'opère par exemple par empâtage de poudre de zéolite avec une pâte argileuse, dans des proportions de l'ordre de 80 à 90% en poids de poudre de zéolite pour 20 à 10% en poids de liant d'agglomération, puis mise en forme en billes, plaquettes ou extrudés, et traitement thermique à haute température pour cuisson de l'argile et réactivation de la zéolite, l'échange au baryum pouvant être effectué avant et/ou après l'agglomération de la zéolite pulvérulente avec le liant d'agglomération.

On obtient des agglomérés zéolitiques dont la granulométrie est en général de quelques millimètres, et qui, si le choix du liant et la granulation sont faits dans les règles de l'art, présentent un ensemble de propriétés satisfaisantes, en particulier de porosité, de résistance mécanique, de résistance à l'abrasion.

Cependant, les propriétés d'adsorption de ces agglomérés sont évidemment réduites par rapport à la poudre active de départ, en raison de la présence de liant d'agglomération inerte.

Divers moyens ont été proposés pour pallier cet inconvénient du liant d'être inerte quant aux performances adsorbantes, parmi lesquels, la transformation du liant, pour tout ou partie, en zéolite ou zéolitisation. Pour effectuer facilement cette opération, on utilise des liants zéolitisables appartenant à la famille de la kaolinite, de l'halloysite, des kaolins et/ou des métakaolins, préalablement calcinés à des températures comprises entre 500°C et 700°C.

Une variante consiste à mouler des grains de kaolin puis à zéolitiser le kaolin ; le principe en est exposé dans « Zeolite Molecular Sieves » de D.W. BRECK, John Wiley and Sons, New York, p 320 et suivantes ». Cette technologie a été appliquée avec succès pour l'obtention de grains de zéolite A ou de zéolite X, constitués jusqu'à 95% en poids de la zéolite elle-même et d'un résiduel de liant non transformé (voir par exemple US 3 119 660 où on constate que l'obtention de zéolite X nécessite l'ajout d'une source de silice dans le milieu réactionnel).

Dans US 4 818 508 sont préparés des agglomérés à base de zéolite A, X ou Y par digestion de préformes d'argile réactive (obtenue par traitement thermique d'argile non-réactive - telle que halloysite ou kaolinite- dont au moins 50% en poids se présente sous forme de particules de granulométrie comprise entre 1,5 $\mu$m et 15 $\mu$m), de préférence en présence d'agent porogène et avec un oxyde de métal alcalin. Les exemples relatifs à la synthèse d'agglomérés à base de zéolite X montrent là aussi qu'il est nécessaire d'ajouter une source de silice, ce qui n'est pas le cas pour préparer des agglomérés à base de zéolite A. Cependant, ce procédé ne permet pas a priori de contrôler la taille des cristaux de zéolite formés après digestion de l'argile réactive.

JP 3 066 430 enseigne que l'on peut former des grains de zéolite X ayant un rapport Si/Al faible, inférieur à 1,25, en mélangeant une poudre de zéolite LSX de rapport Si/Al inférieur à 1,25 avec du kaolin, de la potasse, de la soude et de la carboxyméthylcellulose, suivi d'une mise en forme par extrusion. Les grains ainsi obtenus sont séchés, calcinés à 600°C pendant 2 heures puis immergés dans une solution de soude et de potasse à 400°C pendant 2 jours.

Néanmoins, les procédés associés décrits dans les brevets précédents sont lourds et pèchent, soit par la durée excessive de réaction, soit par le nombre important d'étapes mises en jeu. Selon les inventeurs, on peut, en outre, craindre que le traitement thermique tel que décrit et revendiqué dans JP 3 066 430, après l'étape de mise en forme, ne contribue à l'amorphisation des grains et que la digestion caustique qui suit ait pour objet de les recristalliser, ce qui expliquerait la lenteur du procédé.

US 6 410 815 décrit un procédé de fabrication d'agglomérés de zéolite LSX de rapport Si/Al tel que 1 $\leq$ Si/Al $\leq$ 1,15 échangés au baryum et éventuellement au potassium, en agglomérant de la poudre de zéolite LSX avec un liant qui peut être éventuellement zéolitisé par immersion de l'aggloméré dans une liqueur alcaline. Après échange des ions de la zéolite par des ions baryum (et éventuellement potassium) et activation, les agglomérés ainsi obtenus présentent des sélectivités vis-à-vis du paraxylène contenu dans les coupes aromatiques en $C_8$ constituées typiquement de paraxylène, métaxylène, orthoxylène, éthylbenzène, améliorées par rapport à des adsorbants préparés à partir de poudre de zéolite X de rapport Si/Al tel que 1,15 < Si/Al $\leq$ 1,5.

Outre de bonnes propriétés de sélectivité vis à vis de l'espèce à séparer du mélange réactionnel, l'adsorbant doit présenter de bonnes propriétés de transfert de matière afin de garantir un nombre de plateaux théoriques suffisants pour réaliser une séparation efficace des espèces en mélange, comme l'indique RUTHVEN dans l'ouvrage intitulé «Principles of Adsorption and Adsorption Processes» («Principes de l'Adsorption et des procédés d'adsorption») pages 326 et 407. RUTHVEN indique, page 243, que, dans le cas d'un adsorbant aggloméré, le transfert de matière global dépend de l'addition de la résistance diffusionnelle intra-cristalline et de la résistance diffusionnelle entre les cristaux. La résistance diffusionnelle intra-cristalline est proportionnelle au carré des rayons des cristaux et inversement proportionnelle à la diffusivité des molécules intra-cristalline. La résistance diffusionnelle entre les cristaux (également appelée résistance macroporeuse) est quant à elle proportionnelle au carré des rayons des agglomérés et inversement proportionnelle à la diffusivité des molécules dans les macropores. Pour une structure zéolitique, une taille d'aggloméré et une température de fonctionnement données, les diffusivités sont fixées et le seul moyen d'améliorer le transfert de matière consiste à réduire le diamètre des cristaux. Un gain sur le transfert global sera donc obtenu en réduisant la taille des cristaux.

Pour estimer cette amélioration de la cinétique de transfert, on peut utiliser la théorie des plateaux décrite par RUTHVEN dans «Principles of Adsorption and Adsorption processes » chapitre 8, pages 248-250. Cette approche est basée sur la représentation d'une colonne par un nombre fini de réacteurs hypothétiques idéalement agités (étages théoriques). La hauteur équivalente de plateau théorique est une mesure directe de la dispersion axiale et de la résistance au transfert de matière du système.

Dans le cas particulier de la purification des gaz, l'art antérieur (EP 1 105 213) a reconnu qu'une diminution de la taille des cristaux augmentait la capacité d'adsorption en fonctionnement dynamique et diminuait la résistance à la diffusion intra-cristalline. EP 1 105 213 décrit à cet effet un procédé de fabrication de tamis moléculaire pour l'élimination de $CO_2$. Ce tamis moléculaire est formé en agglomérant une poudre de faujasite X à faible teneur en silice (LSX) dont plus de 97% des sites cationiques sont occupés par des ions sodium, avec un liant inerte, un peptisant et un agent porogène. Entre autre, plus de 80% de ladite poudre LSX doit être constituée de cristaux compris entre 1 $\mu$m et 2 $\mu$m afin d'améliorer la capacité d'adsorption en dynamique du $CO_2$ et de diminuer la résistance au transfert intra-cristallin.

Une troisième propriété de l'adsorbant nécessaire pour garantir de bonnes performances du procédé de séparation en phase liquide de type contre-courant simulé est une bonne résistance mécanique. En effet, en condition standard de fonctionnement de ce type de procédé, une contrainte mécanique s'applique sur l'adsorbant au sein des unités, entraînant la formation de fines, qui induisent une détérioration des performances (« Primary analysis on state of xylene adsorption unit », Li et coll., Jingxi Shiyou Huagong, 4, (2004), 54-55), et ce d'autant plus que la résistance mécanique de l'adsorbant sera faible.

La résistance mécanique des adsorbants est caractérisée à l'aide d'un appareil « BCS Tester » commercialisé par la société Vinci Technologies selon la méthode (fournie avec le dispositif) Shell série SMS1471-74 (Shell Method Series SMS1471-74 « Determination of Bulk Crushing Strength Of Catalysts ». Compression-Sieve Method) qui permet de déterminer la résistance à l'écrasement d'un lit de solides (billes ou extrudés de longueur inférieure ou égale à 6 mm).

Ces tests montrent que toutes choses étant égales par ailleurs, un adsorbant est d'autant moins résistant mécaniquement que la taille des cristaux qui le constituent est faible.

Il est connu que les adsorbants zéolitiques agglomérés à base de zéolite LSX échangée au baryum ou (baryum + potassium) présentent de bonnes propriétés d'adsorption des xylènes, une bonne sélectivité du paraxylène dans un mélange de $C_8$ aromatiques en phase liquide.

Il est également connu que les petits cristaux de zéolite apportent en général un meilleur transfert de matière que les cristaux de la même zéolite de granulométrie supérieure, notamment du fait du transfert de matière amélioré.

L'homme du métier s'attend donc à ce que des adsorbants zéolitiques agglomérés à base de zéolite LSX à petits cristaux et échangés au baryum ou (baryum + potassium) présentent de bonnes propriétés d'adsorption du paraxylène, une bonne sélectivité et un bon transfert de matière et donc à ce que de tels adsorbants présentent de bonnes performances de séparation du paraxylène contenu dans un mélange de $C_8$ aromatiques dans un procédé en phase liquide, par exemple de type contre-courant simulé.

Cependant, les inventeurs ont constaté que des adsorbants zéolitiques à base de zéolite LSX à petits cristaux agglomérés avec un liant de manière classique n'ont pas une bonne résistance mécanique et que leurs performances de séparation des isomères aromatiques en C8 et notamment des xylènes se dégradent au cours du temps du fait de la formation de fines, et ce d'autant plus vite que la taille des cristaux de zéolite LSX est faible.

Il a maintenant été découvert qu'il est possible de préparer des adsorbants zéolitiques à partir de cristaux de zéolite LSX de granulométrie faible, tout en conservant une résistance mécanique élevée des agglomérés, et notamment une résistance satisfaisante pour les applications de séparation, notamment des xylènes, en particulier du paraxylène.

La présente invention a pour objet des adsorbants zéolitiques, utilisables notamment pour la séparation du paraxylène d'un mélange de composés aromatiques en $C_8$, présentant d'excellentes performances, en particulier en terme de sélectivité vis-à-vis du paraxylène, de capacité d'adsorption des xylènes, de transfert de matière et de résistance mécanique, lesdits adsorbants étant particulièrement adaptés pour une utilisation dans un procédé de séparation du paraxylène en phase liquide, de préférence de type contre-courant simulé.

Plus précisément, l'adsorbant zéolitique aggloméré selon l'invention est à base de cristaux de zéolite LSX de diamètre moyen en nombre compris entre 0,1 $\mu$m et 4 $\mu$m, de rapport atomique Si/Al = 1,00 ± 0,05, dont au moins 90% des sites cationiques échangeables sont occupés soit par des ions baryum seuls soit par des ions baryum et des ions potassium, caractérisé en ce que la résistance mécanique mesurée par la méthode Shell série SMS1471-74 adaptée aux agglomérés de taille inférieure à 1,6 mm est supérieure ou égale à 2 MPa.

La résistance mécanique indiquée ci-dessus est mesurée à l'aide d'un appareil « BCS Tester » commercialisé par la société Vinci Technologies selon la méthode Shell série SMS1471-74, et adaptée aux agglomérés zéolitiques de taille inférieure à 1,6 mm.

Selon un mode de réalisation préféré de l'invention, l'adsorbant zéolitique aggloméré présente en outre une ou plusieurs des caractéristiques suivantes, prises isolément ou en combinaison :

- l'adsorbant zéolitique aggloméré comprend de la poudre de zéolite LSX ayant un rapport atomique Si/Al = 1,00 ± 0,05, dont les valeurs inférieures à l'unité traduisent les incertitudes analytiques sur la mesure de ce rapport, et les valeurs supérieures, soit la même incertitude analytique, soit un écart tolérable de pureté du produit, échangée à au moins 90% par des ions baryum seuls soit par des ions baryum et des ions potassium, les sites échangeables occupés par le potassium pouvant représenter jusqu'à 1/3 des sites échangeables occupés par les ions baryum + potassium (le complément éventuel étant généralement assuré par des ions alcalins ou alcalino-terreux autres que le baryum et le potassium) ;
- l'adsorbant zéolitique aggloméré comprend un liant inerte au sens de l'adsorption en proportion inférieure ou égale à 20% en poids, de préférence 15% en poids, de la masse totale de l'aggloméré ;
- le volume de Dubinin de l'adsorbant zéolitique aggloméré, qui est une estimation du volume microporeux mesuré par adsorption d'azote à 77 K, est supérieur ou égal à 0,200 cm$^3$/g et de préférence supérieur ou égal à 0,220 cm$^3$/g et de préférence supérieur ou égal à 0,225 cm$^3$/g.

Les adsorbants zéolitiques agglomérés de l'invention présentent généralement une distribution de taille donnant un diamètre moyen compris entre 0,3 mm et 1,6 mm.

La distribution de la taille des granulés est mesurée par granulométrie laser en voie liquide à l'aide d'un appareil Mastersizer 2000.

La technique de granulométrie laser utilise le principe de diffraction et de diffusion d'un faisceau laser (466 et 633 nm de longueur d'onde) créées par le passage des granulés dans son champ optique. Elle repose sur les deux théories fondamentales de Fraunhofer et de Mie.

La mesure de la distribution de taille d'un échantillon sur le Mastersizer 2000 comporte trois étapes :

- l'échantillon est préalablement dispersé dans de l'eau déminéralisée jusqu'à obtention de la concentration requise avant d'être introduit dans le banc optique ;

- l'image de diffraction de l'échantillon est saisie sur le banc optique en faisant circuler l'échantillon à travers une cellule de verre à faces parallèles éclairée par un faisceau de lumière laser (466 et 633 nm) ;
- les données brutes sont analysées à l'aide du logiciel Malvern.

Le résultat obtenu est la distribution granulométrique où l'abscisse (en $\mu$m) correspond au diamètre des granulés et où l'ordonnée représente le pourcentage volumique de chaque classe granulométrique, distribution dont on peut déduire le diamètre médian en volume D(v, 0,5).

Le volume de Dubinin est calculé selon la relation de Dubinin-Radushkevich, telle que décrite par Lowell et coll. dans « Characterization of Porous Solids and Powders : Surface Area, Pore Size and Density », chapitre 9, « Micropore Analysis », pages 143-145 :

$$\log V = \log V_0 - D(\log \frac{P}{P_0})^2$$

qui relie le volume V d'azote adsorbé dans le matériau adsorbant à la pression relative P / $P_0$. Le volume de Dubinin est le volume $V_0$, volume maximal de vapeur d'azote que l'on peut condenser dans les micropores du matériau adsorbant. Il s'exprime en $cm^3$ de vapeur d'azote (ramenée aux conditions normales) par gramme d'adsorbant.

Le volume de Dubinin est alors calculé à partir du volume $V_0$ de gaz, qui est ensuite converti en volume de liquide : il s'exprime en $cm^3$ par gramme d'adsorbant, et correspond au volume microporeux disponible pour l'adsorption.

Préalablement à la mesure, l'échantillon est prétraité à 500°C pendant 12 heures sous vide (P < 5.10$^{-6}$ Torr; soit 6,7.10$^{-4}$ Pa). La mesure est ensuite effectuée sur un appareil de type ASAP 2010 M de Micromeritics. Le tracé de l'isotherme est réalisé à l'aide d'une table de pression d'au moins 35 points entre 0,01 et 1 P/$P_0$. On porte sur un diagramme la valeur de log V en fonction de $(\log(P/P_0))^2$. Le volume de Dubinin est obtenu à partir de l'ordonnée à l'origine de la droite de régression linéaire des points dont $(\log(P/P_0))^2$ est compris entre 1 et 2 (soit 0,039 < P/$P_0$ < 0,1). L'incertitude de mesure est de $\pm$ 0,003.

Selon un mode de réalisation préféré, la zéolite LSX des agglomérés selon l'invention est constituée essentiellement de cristaux de diamètre moyen (en nombre) mesuré par MEB et comptage, compris au sens large entre 0,1 $\mu$m et 4 $\mu$m, préférentiellement compris entre 0,1 $\mu$m et 3 $\mu$m, et avantageusement compris entre 0,1 $\mu$m et 2 $\mu$m.

Par liant au sens de la présente invention, on entend une matrice inorganique inerte comprenant des matériaux amorphes tels que de la silice, des mélanges de silice et d'alumine et/ou des composés tels que des argiles. On ne sortirait pas du cadre de la présente invention si cette matrice contenait des matériaux cristallins zéolitiques autres que de la zéolite LSX telles que définie précédemment en quantité ne dépassant pas 5% du poids total de l'aggloméré.

L'invention concerne également un procédé de préparation des agglomérés selon l'invention qui comprend les étapes suivantes:

a) agglomération de cristaux de zéolite LSX, avec un liant d'agglomération, qui contient de préférence au moins 80% en poids d'argile(s) zéolitisable(s) (partie zéolitisable), et éventuellement une source de silice, puis mise en forme, séchage et calcination de la poudre agglomérée,

b) zéolitisation de ladite partie zéolitisable du liant par action d'une solution alcaline basique,

c) remplacement d'au moins 90% des sites échangeables du produit obtenu à l'étape b) par du baryum, suivi du lavage et du séchage du produit ainsi traité (on entend par sites échangeables de l'aggloméré, l'ensemble des sites échangeables de la poudre de zéolite LSX et ceux formés par la zéolitisation éventuelle du liant), d) éventuellement remplacement d'au plus 33% des sites échangeables du produit obtenu à l'étape c) par du potassium, suivi du lavage et du séchage du produit ainsi traité, et

e/ activation éventuelle du produit obtenu,

étant entendu que l'échange éventuel au potassium (étape d)) peut être pratiqué avant et/ou après l'échange au baryum (étape c)).

De manière préférentielle, le procédé de préparation selon l'invention est constitué essentiellement des étapes a) à c)/ puis e), ou a) à e), définies précédemment.

Dans l'étape a), le liant d'agglomération est de préférence présent en proportion inférieure ou égale à 20% en poids par rapport à l'aggloméré total.

La taille des cristaux de zéolite LSX utilisés à l'étape a) et des cristaux de zéolite LSX contenus dans les agglomérés est mesurée par observation et comptage au microscope électronique à balayage (MEB).

L'observation au MEB permet également de confirmer la présence de liant inerte au sein des agglomérés.

Les cristaux de zéolite LSX de diamètre moyen en nombre compris entre 0,1 $\mu$m et 4 $\mu$m mis en œuvre dans le cadre de la présente invention sont considérés comme des petits cristaux ; les cristaux de zéolite X commerciaux les plus usuels présentant un diamètre en général supérieur à 7 $\mu$m.

L'agglomération et la mise en forme (étape a)) peuvent être réalisées selon toutes les techniques connues de l'homme de l'art, telles qu'extrusion, compactage, agglomération.

Le liant d'agglomération a essentiellement pour rôle de faciliter la mise en forme et l'agglomération des poudres de zéolites. De manière préférentielle, le liant est inerte au sens de l'adsorption. Le liant d'agglomération mis en oeuvre contient une partie zéolitisable, i. e. une ou plusieurs argile(s) zéolitisable(s), de préférence de 80% à 100% du poids total de liant, et peut également contenir d'autres liants minéraux tels que bentonite, attapulgite, sépiolite.

Par argile zéolitisable, on entend une argile ou un mélange d'argiles qui sont susceptibles de se transformer en matière zéolitique par action d'une solution basique alcaline.

Les argiles zéolitisables appartiennent en général à la famille de la kaolinite, de l'halloysite, des nacrites, des dickites, des kaolins et/ou des métakaolins auxquelles on peut ajouter une source de silice. Le kaolin est couramment utilisé. La calcination qui suit le séchage est menée à une température en général comprise entre 500 et 600°C.

De plus, lors de l'étape a), outre la poudre de zéolite et le liant d'agglomération, des additifs peuvent également être mis en œuvre, par exemple des agents porogènes et/ou des additifs destinés à faciliter l'agglomération et/ou à améliorer le durcissement des agglomérés formés.

Les agglomérés issus de l'étape a), qu'ils soient sous forme de billes, d'extrudés ont en général un diamètre moyen en nombre allant de 0,4 à 2 mm, et en particulier entre 0,4 et 1,6 mm, plus particulièrement entre 0,4 et 0,8 mm.

Dans le présent document, on emploie l'appellation « diamètre moyen en nombre » ou bien « taille » pour les cristaux de zéolite et pour les agglomérés zéolitiques. La précision est de l'ordre de 3%.

A l'issue de l'étape a), les particules d'agglomérés les plus fines peuvent être éliminées par cyclonage et/ou tamisage et/ou les particules trop grosses par tamisage ou concassage, dans le cas d'extrudés, par exemple.

La poudre de zéolite LSX mise en œuvre à l'étape a) peut être issue de la synthèse de cristaux de zéolite sous forme NaKLSX, mais on ne sortirait pas du cadre de l'invention en utilisant une poudre ayant subi un ou plusieurs échanges cationiques, entre la synthèse sous forme NaKLSX et sa mise en œuvre à l'étape a).

Grâce à l'étape b) de zéolitisation, on obtient la transformation, d'au moins 50% et de préférence d'au moins 70 à 82% en poids de la ou des argile(s) zéolitisable(s) contenue(s) dans le liant en matière zéolitique ; on constate que la zéolitisation permet notamment de renforcer la résistance mécanique des adsorbants zéolitiques agglomérés.

La zéolitisation peut être pratiquée par immersion de l'aggloméré dans une solution basique alcaline, en général aqueuse, par exemple une solution aqueuse de soude et/ou de potasse, dont la concentration est de préférence supérieure à 0,5 M. Ladite concentration est généralement inférieure à 3 M, de préférence inférieure à 2 M, avantageusement inférieure à 1 M. La zéolitisation s'opère de préférence à chaud (température supérieure à la température ambiante) typiquement à des températures de l'ordre de 80-100°C, afin d'améliorer la cinétique du processus et de réduire les durées d'immersion à moins de 8 heures mais on ne sortirait pas du cadre de l'invention en opérant à des températures plus basses et des durées d'immersion plus longues.

Selon ce mode opératoire, on obtient aisément la zéolitisation d'au moins 50%, et de préférence d'au moins 70 à 82% en poids de la ou des argile(s) zéolitisable(s) contenue(s) dans le liant. On procède ensuite à un lavage à l'eau suivi d'un séchage.

L'étape c) d'échange au baryum des cations de la zéolite s'effectue par mise en contact des agglomérés issus de l'étape b) (ou d)) avec un sel de baryum, tel que $BaCl_2$, en solution aqueuse à une température comprise entre la température ambiante et 100°C, et de préférence comprise entre 80 et 100°C. Pour obtenir rapidement un taux d'échange en baryum élevé, i. e. supérieur à 90%, on préfère opérer avec un large excès de baryum par rapport aux cations de l'aggloméré que l'on souhaite échanger, typiquement tel que le rapport Ba/Al soit de l'ordre de 5 à 6 en procédant par échanges successifs de façon à atteindre le taux d'échange visé minimum d'au moins 90% et de préférence d'au moins 95%. Dans tout le texte les taux d'échange sont calculés en équivalent et non en molarité.

L'échange éventuel au potassium (étape d)) peut être pratiqué avant et/ou après l'échange au baryum (étape c)) ou de manière simultanée en utilisant une solution contenant les ions baryum et potassium. Comme indiqué précédemment, il est également possible d'agglomérer à l'étape a) de la poudre de zéolite LSX contenant déjà des ions potassium et s'affranchir (ou non) de l'étape d).

L'activation (étape e)), dernière étape du procédé d'obtention des adsorbants selon l'invention, a pour but de fixer la teneur en eau, ainsi que la perte au feu de l'adsorbant dans des limites optimales. On procède en général par activation thermique qu'on exécute préférentiellement entre 200°C et 300°C pendant un certain temps, typiquement de 1 à 6 heures, en fonction de la teneur en eau et de la perte au feu souhaitées et selon l'utilisation de l'adsorbant qui est visée.

L'invention concerne également les utilisations des adsorbants zéolitiques décrits ci-dessus comme agents d'adsorption susceptibles de remplacer avantageusement les agents d'adsorption décrits dans la littérature à base de zéolite X ou à base de zéolite LSX, échangée au baryum ou échangée au baryum et potassium, et notamment dans les utilisations listées ci-dessous :

- la séparation des isomères aromatiques en $C_8$ et notamment des xylènes,
- la séparation de sucres,
- la séparation d'alcools polyhydriques,
- la séparation d'isomères de toluène substitué tels que nitrotoluène, diéthyltoluène, toluènediamine,
- la séparation des crésols,
- la séparation des dichlorobenzènes.

L'invention concerne notamment un perfectionnement de procédé de récupération de paraxylène à partir de coupes d'isomères $C_8$ aromatiques en utilisant, comme agent d'adsorption du paraxylène, un adsorbant zéolitique aggloméré selon l'invention mis en oeuvre dans des procédés en phase liquide mais aussi en phase gazeuse.

L'invention concerne particulièrement un procédé de production de paraxylène à haute pureté à partir d'une charge d'hydrocarbures aromatiques contenant des isomères à 8 atomes de carbones comprenant les étapes suivantes :

a) Une étape de mise en contact, dans des conditions d'adsorption adéquates, de la charge avec un lit d'adsorbant selon l'invention, de manière à adsorber préférentiellement le paraxylène,
b) une étape de mise en contact, dans des conditions de désorption, du lit d'adsorbant avec un désorbant, qui est préférentiellement soit du toluène, soit du paradiéthylbenzène,
c) une étape de soutirage du lit d'adsorbant d'un flux contenant le désorbant et les produits de la charge les moins sélectivement adsorbés,
d) une étape de soutirage du lit d'adsorbant d'un flux contenant le désorbant et le paraxylène,
e) une séparation du flux issu de l'étape c) en un premier flux contenant le désorbant et un second flux contenant les produits de la charge les moins sélectivement adsorbés,
f) une séparation du flux issu de l'étape d) en un premier flux contenant le désorbant et un second flux contenant du paraxylène à un niveau de pureté supérieure ou égale à 75% et de préférence supérieure ou égale à 99,7%.

Le procédé peut également optionnellement inclure les étapes suivantes :

g) une étape de cristallisation dans un cristalliseur consistant en la cristallisation du paraxylène issu de l'étape f), permettant d'obtenir d'une part des cristaux de paraxylène imbibés de leur liqueur mère, et d'autre part une liqueur mère qui peut être en partie, voire en totalité, recyclée en mélange avec la charge fraîche à l'entrée de l'unité d'adsorption en lit mobile simulé,
h) une étape de lavage des cristaux issus de l'étape g) à l'issue de laquelle on récupère du paraxylène à une pureté d'au moins 99,7%, et de manière préférée d'au moins 99,8%.

On peut ainsi séparer le produit désiré par chromatographie liquide d'adsorption préparative (en batch), avantageusement en lit mobile simulé, c'est-à-dire à contre-courant simulé ou à co-courant simulé, et plus particulièrement à contre-courant simulé.

La séparation chromatographique en lit mobile simulé à contre-courant simulé est bien connue dans l'état de la technique. En règle générale, une unité de séparation en lit mobile simulé comprend au moins une colonne d'adsorption contenant une pluralité de lits d'un adsorbant, interconnectés en boucle fermée. L'unité de séparation en lit mobile simulé comporte au moins trois zones chromatographiques, et éventuellement quatre ou cinq, chacune de ces zones étant constituée par au moins un lit ou une portion de colonne et comprise entre deux points successifs d'alimentation ou soutirage. Typiquement, on alimente au moins une charge à fractionner et un désorbant (parfois appelé éluant) et l'on soutire au moins un raffinat et un extrait. Les points d'alimentation et de soutirage sont modifiés au cours du temps, typiquement décalés vers le bas d'un lit et ce de façon synchrone.

Par définition, on désigne chacune des zones de fonctionnement par un numéro :

- Zone 1 = zone de désorption du produit recherché (contenu dans l'extrait) comprise entre l'injection du désorbant et le prélèvement de l'extrait;
- Zone 2 = zone de désorption des composés du raffinat, comprise entre le prélèvement de l'extrait et l'injection de la charge à fractionner ;
- Zone 3 = zone d'adsorption du produit recherché, comprise entre l'injection de la charge et le soutirage du raffinat, et
- Zone 4 située entre le soutirage de raffinat et l'injection du désorbant.

Les conditions opératoires d'une unité industrielle d'adsorption de type contre-courant simulé sont en général les suivantes :

- nombre de lits 6 à 30

- nombre de zones au moins 4
- température 100 à 250°C, de préférence 150 à 190°C
- pression comprise entre la pression de bulle des xylènes à la température du procédé et 3 MPa
- rapport des débits désorbant sur charge 0,7 à 2,5 (par exemple 0,9 à 1,8 pour une unité d'adsorption seule (stand alone) et 0,7 à 1,4 pour une unité d'adsorption combinée à une unité de cristallisation)
- taux de recyclage de 2,5 à 12, de préférence 3,5 à 6.

On pourra se référer à l'enseignement des brevets US 2 985 589, US 5 284 992 et US 5 629 467.

Les conditions opératoires d'une unité industrielle d'adsorption à co- courant simulé sont en général les mêmes que celles fonctionnant à contre- courant simulé à l'exception du taux de recyclage qui est en général compris entre 0,8 et 7. On pourra se référer aux brevets US 4 402 832 et US 4 498 991.

Le solvant de désorption peut être un désorbant dont le point d'ébullition est inférieur à celui de la charge, tel que le toluène mais aussi un désorbant dont le point d'ébullition est supérieur à celui de la charge, tel que le paradiéthylbenzène (PDEB). La sélectivité des adsorbants selon l'invention pour l'adsorption du paraxylène contenu dans des coupes aromatiques en $C_8$ est optimale lorsque leur perte au feu mesurée à 900°C est comprise en général entre 4,0 et 7,7%, et de préférence entre 4,7 et 6,7%. De l'eau et un peu de dioxyde de carbone entrent dans la perte au feu.

Une des techniques de choix pour caractériser l'adsorption de molécules en phase liquide sur un solide poreux est de réaliser un perçage. Dans son ouvrage « Principles of Adsorption and Adsorption processes » (« Principes de l'Adsorption et des procédés d'adsorption »), Ruthven définit la technique des courbes de perçage (« breakthrough curves ») comme l'étude de l'injection d'un échelon de constituants adsorbables. Cette technique permet d'accéder simultanément (à la fois) aux propriétés de sélectivité du solide poreux vis à vis des molécules à séparer, ainsi qu'aux propriétés de transfert de matière.

Les agglomérés de la présente invention présentent un diamètre moyen compris entre 0,3 mm et 1,6 mm et à ce jour il n'existe pas de méthode permettant de caractériser la résistance à l'écrasement en lit d'agglomérés de diamètre moyen aussi faible.

La méthode Shell série SMS1471-74 (Shell Method Series SMS1471-74 « Determination of Bulk Crushing Strength Of Catalysts ». Compression-Sieve Method) associée à l'appareil « BCS Tester » commercialisé par la société Vinci Technologies et qui permet de déterminer la résistance à l'écrasement d'un lit de solides est basé sur le principe suivant : un échantillon de 20 cm$^3$ d'adsorbants agglomérés, préalablement séché à l'étuve pendant au moins 2 heures à 250°C, est placé dans un cylindre métallique de section interne connue. Une force croissante est imposée par paliers par l'intermédiaire d'un piston sur cet échantillon.

Les fines obtenues aux différents paliers de pression sont séparées par tamisage et pesées. Le tamis utilisé dans la méthode Shell standard est de 425 $\mu$m ce qui doit être adapté pour les agglomérés de taille inférieure à 1,6 mm pour lesquels on utilise un tamis de 200 $\mu$m. La résistance à l'écrasement en lit est déterminée par la pression en méga Pascal (MPa) pour laquelle la quantité de fines cumulées passant à travers le tamis s'élève à 0,5% pondéral de l'échantillon.

Cette valeur est obtenue en traçant sur un graphique la masse de fines obtenue en fonction de la force appliquée sur le lit d'adsorbant et en interpolant à 0,5% massique de fines cumulées. La résistance à l'écrasement en lit est typiquement comprise entre quelques centaines de kPa et quelques dizaines de MPa et généralement comprise entre 0,3 et 3,2 MPa.

La présente invention est maintenant décrite à l'aide des exemples qui suivent, qui ont pour but d'illustrer certains modes de réalisation de l'invention, sans toutefois limiter la portée de ladite invention telle qu'elle est revendiquée dans les revendications annexées.

## Procédé de synthèse de poudre de zéolite LSX (Synthèse A)

Étape a) : Préparation d'une Solution S1 d'aluminate de sodium et de potassium

On réalise une solution contenant :

- eau déminéralisée : 800 g
- hydroxyde de sodium (pureté 99%) : 420 g
- hydroxyde de potassium (pureté 85%) : 255 g

Cette solution est portée à 115 °C puis on ajoute 240 g d'hydroxyde d'aluminium (type gibbsite).

Étape b) : Préparation d'une Solution S2 de silicate de sodium

On réalise une solution contenant :

- eau déminéralisée : 620 g
- silicate de sodium : 710 g

Étape c) : Préparation du gel

Dans un réacteur de 3 litres muni d'un agitateur type vis d'Archimède, on mélange les solutions S1 et S2 au moyen d'une turbine défloculeuse à 2000 tours/minute pendant 5 minutes pour obtenir un gel homogène. Cette composition correspond à la stœchiométrie suivante :
$3,95\ Na_2O$ ; $1,26\ K_2O$ ; $2\ SiO_2$ ; $1\ Al_2O_3$ ; $77,4\ H_2O$.
Après mélange des réactifs et gélification, le gel mûrit ensuite pendant 18 heures à 50°C, puis on effectue une cristallisation pendant 4 heures à 95°C. Le mûrissement et la cristallisation sont effectués sous agitation à une vitesse de 200 tours/minute.
Les cristaux obtenus après filtration, lavage et séchage sont identifiés par diffraction des rayons X comme faujasites. L'analyse chimique du solide donne un rapport Si/Al = 1,01.
Le volume microporeux mesuré selon la méthode de Dubinin par adsorption d'azote à 77°K après prétraitement à 500°C pendant 12 heures sous vide est de 0,308 $\pm$ 0,003 $cm^3$/g, soit un taux de cristallinité de 95,5%.
L'analyse de la taille des cristaux de zéolite est réalisée par microscopie électronique à balayage. La taille moyenne des cristaux est de 2,6 $\mu$m.

**Exemple 1 : (comparatif, selon l'enseignement général)**

840 g (exprimés en équivalent calciné) d'une poudre de zéolite LSX de rapport Si/Al = 1,01 obtenue selon le processus décrit dans le brevet européen EP 0 486 384 ou le brevet US 5 173 462, est agglomérée en la mélangeant intimement avec 160 g de kaolinite des Charentes (exprimés en équivalent calciné) avec une quantité d'eau adéquate pour assurer la mise en forme d'agglomérés par extrusion. Les extrudés sont séchés, concassés de manière à récupérer des agglomérés dont le diamètre équivalent est égal à 0,7 mm, puis calcinés à 600°C sous courant d'azote pendant 2 heures.
Ces agglomérés sont échangés au moyen d'une solution de chlorure de baryum 0,5 M à 95°C en 4 étapes. A chaque étape, le rapport volume de solution sur masse de solide est de 20 mL/g et l'échange est poursuivi pendant 4 heures à chaque fois. Entre chaque échange, le solide est lavé plusieurs fois de manière à le débarrasser des excédents de sel. Il est ensuite activé à une température de 200°C pendant 2 heures sous courant d'azote.
Le taux d'échange en baryum est de 94,7% et la perte au feu (mesurée à 900°C) est de 6,6%. Le volume microporeux mesuré selon la méthode de Dubinin par adsorption d'azote à 77 K après prétraitement à 500°C pendant 12 heures sous vide est de 0,215 $cm^3$/g.
L'analyse de la taille des cristaux de zéolite est réalisée par microscopie électronique à balayage. La taille moyenne des cristaux est de 7 $\mu$m.
Un test de perçage (chromatographie frontale) est ensuite réalisé sur ces adsorbants pour évaluer leur efficacité. La quantité d'adsorbant utilisée pour ce test est d'environ 82 g.
Le mode opératoire pour obtenir les courbes de perçage est le suivant :

- Remplissage de la colonne par le tamis et mise en place dans le banc de test.
- Remplissage par le solvant à température ambiante.
- Montée progressive à la température d'adsorption sous flux de solvant (5 $cm^3$/min).
- Injection de solvant à 10 $cm^3$/min lorsque la température d'adsorption est atteinte
- Permutation solvant/charge pour injecter la charge (10 $cm^3$/min).
- L'injection de la charge est ensuite maintenue un temps suffisant pour atteindre l'équilibre thermodynamique.
- Collecte et analyse de l'effluent du perçage.

La pression est suffisante pour que la charge reste en phase liquide, soit 1 MPa. La température d'adsorption est de 175°C.
La composition de la charge est la suivante :

- Paraxylène : 45% poids
- Métaxylène : 45% poids

- Iso-octane : 10% poids (celui-ci est utilisé comme traceur pour l'estimation des volumes non-sélectifs et n'intervient pas dans la séparation)

Les résultats du perçage sont consignés dans le Tableau 1 ci-dessous.

La sélectivité du paraxylène par rapport au métaxylène est calculée par bilan matière.

-- **Tableau 1** --

| Nature du solide | PAF[(1)] à 900°C | Temp [(2)] | Capacité [(3)] | Sélectivité[(4)] $\alpha_{PX/MX}$ | Hauteur de plateau théorique |
|---|---|---|---|---|---|
| BaLSX | 6,6% | 175°C | 0,171 | 3,54 | 4,8 cm |
| (1) PAF: Perte au feu<br>(2) Temp. : température d'adsorption<br>(3) La capacité est exprimée en $cm^3$ de C8-aromatiques adsorbé par gramme d'adsorbant<br>(4) PX: paraxylène, MX: métaxylène | | | | | |

On mesure également la résistance mécanique selon la méthode présentée dans la description de l'invention. La pression nécessaire pour obtenir 0,5% de fines est de 2,40 MPa.

**Exemple 2 (comparatif)** :

Dans cet exemple, on réalise et on teste un adsorbant selon l'art antérieur: exemple 2 de US 6 410 815 reproduit à l'identique.

On agglomère 950 grammes (équivalent calciné) d'une poudre de zéolite LSX de rapport Si/Al = 1,01 obtenue selon le processus décrit dans le brevet européen EP 0 486 384 ou le brevet US 5 173 462, avec 170 g (équivalent calciné) de kaolinite des Charentes, 6 g de carboxyméthylcellulose et la quantité d'eau adéquate pour pouvoir extruder correctement la pâte obtenue. Les extrudés (agglomérés) sont ensuite séchés puis calcinés à une température de 600°C pendant 2 heures sous courant d'azote sec. On procède alors à un concassage de manière à ramener le diamètre équivalent des agglomérés à 0,7 mm.

Ces agglomérés sont échangés au moyen d'une solution de chlorure de baryum 0,5 M à 95°C en 4 étapes. A chaque étape, le rapport volume de solution sur masse de solide est de 20 mL/g et l'échange est poursuivi pendant 4 heures à chaque fois. Entre chaque échange, le solide est lavé plusieurs fois de manière à le débarrasser des excédents de sel puis activé thermiquement à une température de 220°C pendant 2 heures sous courant d'azote.

Le taux d'échange en baryum est de 94,9% et la perte au feu est de 5%. Le volume microporeux mesuré selon la méthode de Dubinin par adsorption d'azote à 77 K après prétraitement à 500°C pendant 12 heures sous vide est de 0,208 $cm^3$/g.

L'analyse de la taille des cristaux de zéolite est réalisée par microscopie électronique à balayage. La taille moyenne des cristaux est de 7 $\mu$m.

Un test de perçage (chromatographie frontale) est ensuite réalisé sur ces adsorbants pour évaluer leur efficacité. La quantité d'adsorbant utilisée pour ce test est d'environ 80 g. Le mode opératoire ainsi que la composition de la charge sont identiques à ceux de l'exemple 1.

Les résultats du perçage sont réunis dans le Tableau 2 ci-dessous :

La sélectivité du paraxylène par rapport au métaxylène est calculée par bilan matière.

-- **Tableau 2** --

| Nature du solide | PAF[(1)] à 900°C | Temp[(2)] | Capacité [(3)] | Sélectivité[(4)] $\alpha_{PX/MX}$ | Hauteur de plateau théorique |
|---|---|---|---|---|---|
| BaLSX | 5,0% | 175°C | 0,172 | 3,40 | 11,5 cm |
| (1) PAF: Perte au feu<br>(2) Temp. : température d'adsorption<br>(3) La capacité est exprimée en $cm^3$ de C8-aromatiques adsorbé par gramme d'adsorbant<br>(4) PX: paraxylène, MX: métaxylène | | | | | |

La méthode de mesure de la sélectivité par test de perçage est différente de celle mesurée par test statique, méthode de test utilisée dans le brevet US 6 410 815. Ceci explique pourquoi la sélectivité du paraxylène par rapport au métaxylène calculée dans cet exemple est différente de celle reportée dans l'Exemple 2 de US 6 410 815 bien que l'adsorbant soit identique.

Toutefois, pour comparaison, la mesure de la sélectivité par test statique a été réalisée dans les mêmes conditions que celles décrites dans le brevet US 6 410 815 et donne des résultats similaires à ceux obtenus dans ledit brevet.

On mesure également la résistance mécanique selon la méthode présentée dans la description de l'invention. La pression nécessaire pour obtenir 0,5% de fines est de 2,20 MPa.

Exemple 3 (comparatif) :

840 g (exprimés en équivalent calciné) de poudre de zéolite LSX obtenue selon l'exemple de synthèse A décrit ci-dessus, sont agglomérés en les mélangeant intimement avec 160 g de kaolin (exprimés en équivalent calciné) et la quantité d'eau adéquate pour la mise en forme des agglomérés par extrusion. Les extrudés sont séchés, concassés de manière à récupérer des agglomérés dont le diamètre équivalent est égal à 0,7 mm, puis calcinés à 600°C sous courant d'azote pendant 2 heures.

L'échange baryum est opéré dans des conditions opératoires identiques à celles de l'exemple 1 à l'exception de la concentration de la solution de $BaCl_2$ qui est de 0,7 M suivi d'un lavage puis d'un séchage à 80°C pendant 2 heures et enfin d'une activation à 200°C pendant 2 heures sous courant d'azote.

Le taux d'échange en baryum est de 98% et la perte au feu (mesurée à 900°C) est de 6,3%. Le volume microporeux mesuré selon la méthode de Dubinin par adsorption d'azote à 77 K après prétraitement à 500°C pendant 12 heures sous vide est de 0,205 cm$^3$/g.

L'analyse de la taille des cristaux de zéolite est réalisée par microscopie électronique à balayage. La taille moyenne des cristaux est de 2,6 $\mu$m.

Un test de perçage (chromatographie frontale) est ensuite réalisé sur ces adsorbants zéolitiques agglomérés pour évaluer leur efficacité. La quantité d'adsorbant utilisée pour ce test est d'environ 82 g.

Le mode opératoire ainsi que la composition de la charge sont identiques à ceux de l'exemple 1. Les résultats du perçage sont réunis dans le Tableau 3 ci-dessous.

La sélectivité du paraxylène par rapport au métaxylène est calculée par bilan matière.

-- Tableau 3 --

| Nature du solide | PAF[(1)] à 900°C | Temp[(2)] | Capacité [(3)] | Sélectivité[(4)] $\alpha_{PX/MX}$ | Hauteur de plateau théorique |
|---|---|---|---|---|---|
| BaLSX | 6,3% | 175°C | 0,155 | 4,03 | 2,54 cm |

(1) PAF: Perte au feu
(2) Temp. : température d'adsorption
(3) La capacité est exprimée en cm$^3$ de C8-aromatiques adsorbé par gramme d'adsorbant
(4) PX: paraxylène, MX: métaxylène

On mesure également la résistance mécanique selon la méthode présentée dans la description de l'invention. La pression nécessaire pour obtenir 0,5% de fines est de 1,90 MPa.

Exemple 4 comparatif :

Dans cet exemple, on réalise et on teste un adsorbant selon l'art antérieur.

On agglomère 840 g (exprimés en équivalent calciné) de poudre de zéolite LSX, de rapport Si/Al = 1,01, obtenue selon le processus décrit dans le brevet européen EP 0 486 384 ou le brevet US 5 173 462, avec 160 g de kaolinite des Charentes (exprimés en équivalent calciné) avec la quantité d'eau adéquate pour opérer par extrusion. Les extrudés sont séchés, concassés de manière à récupérer des agglomérés dont le diamètre équivalent est égal à 0,7 mm, puis calcinés à 600°C sous courant d'azote pendant 2 heures.

200 g d'agglomérés ainsi obtenus sont placés dans un réacteur en verre muni d'une double enveloppe régulée à une température de 95 $\pm$ 1°C puis on ajoute 700 mL d'une solution aqueuse de soude de concentration 220 g/L et on laisse le milieu réactionnel sous agitation pendant 3 heures. On procède ensuite au lavage des granulés en 4 opérations successives suivi de la vidange du réacteur. On s'assure de l'efficacité du lavage en mesurant le pH final des eaux de lavage qui doit être compris entre 10 et 10,5.

On procède ensuite à un échange baryum dans des conditions opératoires identiques à celles de l'exemple 1 suivi d'un lavage puis d'un séchage à 80°C pendant 2 heures et enfin d'une activation à 200°C pendant 2 heures sous courant d'azote. Le taux d'échange en baryum de cet adsorbant est de 96% et sa perte au feu est de 6,9%. Le volume microporeux mesuré selon la méthode de Dubinin par adsorption d'azote à 77 K après prétraitement à 500°C pendant 12 heures sous vide est de 0,242 cm$^3$/g.

L'analyse de la taille des cristaux de zéolite est réalisée par microscopie électronique à balayage. La taille moyenne des cristaux est de 7 μm.

Un test de perçage (chromatographie frontale) est ensuite réalisé sur ces adsorbants zéolitiques agglomérés pour évaluer leur efficacité. La quantité d'adsorbant utilisée pour ce test est d'environ 82 g.

Le mode opératoire ainsi que la composition de la charge sont identiques à ceux de l'exemple 1. Les résultats du perçage sont réunis dans le Tableau 4 ci-dessous.

La sélectivité du paraxylène par rapport au métaxylène est calculée par bilan matière.

-- Tableau 4 --

| Nature du solide | PAF[(1)] à 900°C | Temp[(2)] | Capacité [(3)] | Sélectivité[(4)] $\alpha_{PX/MX}$ | Hauteur de plateau théorique |
|---|---|---|---|---|---|
| BaLSX | 6,9% | 175°C | 0,191 | 3,57 | 3,62 cm |
| (1) PAF: Perte au feu<br>(2) Temp. : température d'adsorption<br>(3) La capacité est exprimée en cm$^3$ de C8-aromatiques adsorbé par gramme d'adsorbant<br>(4) PX: paraxylène, MX: métaxylène | | | | | |

On mesure également la résistance mécanique selon la méthode présentée dans la description de l'invention. La pression nécessaire pour obtenir 0,5% de fines est de 2,70 MPa.

**Exemple 5 (comparatif)** :

Dans cet exemple, on réalise et on teste un adsorbant selon l'art antérieur : exemple 3 de US 6 410 815, reproduit à l'identique.

On agglomère 950 grammes d'une zéolite LSX de rapport Si/Al = 1,01, obtenue selon le processus décrit dans le brevet européen EP 0 486 384 ou le brevet US 5 173 462, avec 170 g de kaolinite des Charentes, 6 g de carboxyméthylcellulose et la quantité d'eau adéquate. On extrude. Les extrudés sont séchés, calcinés à une température de 600°C pendant 2 heures sous courant d'azote sec, puis concassés de manière à ramener leur diamètre équivalent à 0,7 mm.

On immerge maintenant 200 g de ces agglomérés dans 340 mL d'une solution de soude à 220 g/L pendant 3 heures à 95°C. On lave successivement quatre fois à l'eau.

Le solide est ensuite échangé au baryum dans les mêmes conditions que celles exposées dans l'exemple 2 suivi d'un lavage puis d'un séchage à 80°C pendant 2 heures et enfin d'une activation à 200°C pendant 2 heures sous courant d'azote.

Le taux d'échange en baryum de cet adsorbant est de 96,5% et sa perte au feu est de 6,6%. Le volume microporeux mesuré selon la méthode de Dubinin par adsorption d'azote à 77 K après prétraitement à 500°C pendant 12 heures sous vide est de 0,237 cm$^3$/g.

L'analyse de la taille des cristaux de zéolite est réalisée par microscopie électronique à balayage. La taille moyenne des cristaux est de 7 μm.

Un test de perçage (chromatographie frontale) est ensuite réalisé sur ces adsorbants pour évaluer leur efficacité. La quantité d'adsorbant utilisée pour ce test est d'environ 82 g.

Le mode opératoire ainsi que la composition de la charge sont identiques à ceux de l'exemple 1.

Les résultats du perçage sont réunis dans le Tableau 5 ci-dessous :

-- Tableau 5 --

| Nature du solide | PAF[(1)] à 900°C | Temp[(2)] | Capacité [(3)] | Sélectivité[(4)] $\alpha_{PX/MX}$ | Hauteur de plateau théorique |
|---|---|---|---|---|---|
| BaLSX | 6,6% | 175°C | 0,189 | 3,61 | 4,80 cm |
| (1) PAF: Perte au feu<br>(2) Temp. : température d'adsorption<br>(3) La capacité est exprimée en cm$^3$ de C8-aromatiques adsorbé par gramme d'adsorbant<br>(4) PX: paraxylène, MX: métaxylène | | | | | |

La méthode de mesure de la sélectivité par test de perçage est différente de celle mesurée par test statique, méthode de test utilisée dans le brevet US 6 410 815. Ceci explique pourquoi la sélectivité du paraxylène par rapport au métaxylène calculée dans cet exemple est différente de celle reportée dans l'exemple 3 de US 6 410 815 bien que l'adsorbant soit identique.

Toutefois, pour comparaison, la mesure de la sélectivité par test statique a été réalisée dans les mêmes conditions que celles décrites dans le brevet US 6 410 815 et donne des résultats similaires à ceux obtenus dans ledit brevet.

On mesure également la résistance mécanique selon la méthode présentée dans la description de l'invention. La pression nécessaire pour obtenir 0,5% de fines est de 2,50 MPa.

## Exemple 6 (selon l'invention) :

On mélange intimement et agglomère 840 g (exprimés en équivalent calciné) de poudre de zéolite LSX obtenue selon l'exemple de synthèse A décrit ci-dessus, 160 g de kaolin (exprimés en équivalent calciné) avec la quantité d'eau adéquate pour opérer par extrusion. Les extrudés sont séchés, concassés de manière à récupérer des grains dont le diamètre équivalent est égal à 0,7 mm, puis calcinés à 600°C sous courant d'azote pendant 2 heures.

200 g de granulés ainsi obtenus sont placés dans un réacteur en verre muni d'une double enveloppe régulée à une température de 95 $\pm$ 1°C puis on ajoute 700 mL d'une solution aqueuse de soude de concentration 220 g/L et laisse le milieu réactionnel sous agitation pendant 3 heures. On procède ensuite au lavage des granulés en 3 opérations successives de lavage à l'eau suivi de la vidange du réacteur.

On s'assure de l'efficacité du lavage en mesurant le pH final des eaux de lavage qui doit être compris entre 10 et 10,5.

On procède ensuite à un échange baryum dans des conditions opératoires identiques à celles de l'exemple 1 suivi d'un lavage puis d'un séchage à 80°C pendant 2 heures et enfin d'une activation à 200°C pendant 2 heures sous courant d'azote.

Le taux d'échange en baryum de cet adsorbant est de 97% et sa perte au feu est de 6,5%. Le volume microporeux mesuré selon la méthode de Dubinin par adsorption d'azote à 77 K après prétraitement à 500°C pendant 12 heures sous vide est de 0,235 cm$^3$/g.

L'analyse de la taille des cristaux de zéolite est réalisée par microscopie électronique à balayage. La taille moyenne des cristaux est de 2,6 $\mu$m.

Un test de perçage (chromatographie frontale) est ensuite réalisé sur ces adsorbants pour évaluer leur efficacité. La quantité d'adsorbant utilisée pour ce test est d'environ 82 g.

Le mode opératoire ainsi que la composition de la charge sont identiques à ceux de l'exemple 1.

Les résultats du perçage sont réunis dans le Tableau 6 ci-dessous :

La sélectivité du paraxylène par rapport au métaxylène est calculée par bilan matière.

-- Tableau 6 --

| Nature du solide | PAF[(1)] à 900°C | Temp[(2)] | Capacité [(3)] | Sélectivité[(4)] $\alpha_{PX/MX}$ | Hauteur de plateau théorique |
|---|---|---|---|---|---|
| BaLSX | 6,5% | 175°C | 0,178 | 3,98 | 2,20 |

(1) PAF: Perte au feu
(2) Temp. : température d'adsorption
(3) La capacité est exprimée en cm$^3$ de C8-aromatiques adsorbé par gramme d'adsorbant
(4) PX: paraxylène, MX: métaxylène

On mesure également la résistance mécanique selon la méthode présentée dans la description de l'invention. La pression nécessaire pour obtenir 0,5% de fines est de 2,70 MPa.

## Exemple 7 (comparatif) :

On mélange intimement et agglomère 840 g (exprimés en équivalent calciné) de poudre de zéolite LSX obtenue selon l'exemple de synthèse A décrit ci-dessus, 52 g de kaolin (exprimés en équivalent calciné) avec la quantité d'eau adéquate pour opérer par extrusion. Les extrudés sont séchés, concassés de manière à récupérer des grains dont le diamètre équivalent est égal à 0,7 mm, puis calcinés à 600°C sous courant d'azote pendant 2 heures.

L'échange baryum est opéré dans des conditions opératoires identiques à celles de l'exemple 1 suivi d'un lavage puis d'un séchage à 80°C pendant 2 heures et enfin d'une activation à 200°C pendant 2 heures sous courant d'azote.

Le taux d'échange en baryum de cet adsorbant est de 97,2% et sa perte au feu est de 6,4%. Le volume microporeux mesuré selon la méthode de Dubinin par adsorption d'azote à 77 K après prétraitement à 500°C pendant 12 heures sous vide est de 0,232 cm$^3$/g.

L'analyse de la taille des cristaux de zéolite est réalisée par microscopie électronique à balayage. La taille moyenne des cristaux est de 2,6 μm.

Un test de perçage (chromatographie frontale) est ensuite réalisé sur ces adsorbants pour évaluer leur efficacité. La quantité d'adsorbant utilisée pour ce test est d'environ 82 g.

Le mode opératoire ainsi que la composition de la charge sont identiques à ceux de l'exemple 1.

Les résultats du perçage sont réunis dans le tableau 7 ci-dessous :

La sélectivité du paraxylène par rapport au métaxylène est calculée par bilan matière.

-- Tableau 7 --

| Nature du solide | PAF[(1)] à 900°C | Temp[(2)] | Capacité [(3)] | Sélectivité[(4)] $\alpha_{PX/MX}$ | Hauteur de plateau théorique |
|---|---|---|---|---|---|
| BaLSX | 6,4% | 175°C | 0,176 | 4,00 | 0,20 cm |

(1) PAF: Perte au feu
(2) Temp. : température d'adsorption
(3) La capacité est exprimée en $cm^3$ de C8-aromatiques adsorbé par gramme d'adsorbant
(4) PX: paraxylène, MX: métaxylène

On mesure également la résistance mécanique selon la méthode présentée dans la description de l'invention. La pression nécessaire pour obtenir 0,5% de fines est de 0,20 MPa.

On constate qu'à capacité d'adsorption identique, un aggloméré qui n'a pas été réalisé suivant le procédé décrit dans la présente invention présente une bonne sélectivité, un bon transfert de matière mais n'a aucune résistance mécanique.

**Exemple 8 (comparatif)** :

On agglomère 950 g d'une zéolite LSX de rapport Si/Al = 1,01 obtenue selon le processus décrit dans le brevet européen EP 0 486 384 ou le brevet US 5 173 462, de taille de cristaux de 7 μm avec 170 g de kaolin et la quantité d'eau adéquate. On extrude. Les extrudés sont séchés, calcinés à une température de 600°C pendant 2 heures sous courant d'azote sec, puis concassés de manière à ramener leur diamètre équivalent à 0,7 mm.

On immerge maintenant 200 g de ces agglomérés dans 340 mL d'une solution de soude à 220 g/L pendant 3 heures à 95°C. On lave successivement quatre fois à l'eau.

On opère ensuite un échange poussé au potassium au moyen d'une solution de KCl 1 M à 25°C en 4 étapes successives.

À chaque étape, le rapport volume de solution sur masse de solide est de 20 mL/g et l'échange est poursuivi pendant 4 heures à chaque fois. Entre chaque échange, le solide est lavé plusieurs fois de manière à le débarrasser des excédents de sel. Le produit obtenu présente un taux d'échange en potassium de 97,5%.

On lui fait alors subir 2 opérations d'échange au baryum identiques à celles décrites à l'exemple 1.

Après toutes ces opérations, le solide est enfin activé à une température de 200°C pendant 2 heures sous courant d'azote.

Il présente les caractéristiques suivantes taux d'échange en baryum 74,3% taux d'échange en potassium 24%.

Le volume microporeux mesuré selon la méthode de Dubinin par adsorption d'azote à 77 K après prétraitement à 500°C pendant 12 heures sous vide est de 0,250 $cm^3$/g et une perte au feu à 900°C de 6,6%.

**Exemple 9** : **adsorbant selon l'invention**

On prépare des échantillons d'agglomérés comme indiqué à l'exemple 8 mais à partir de poudre de zéolite LSX obtenue selon l'exemple de synthèse A décrit ci-dessus.

On procède avant l'échange baryum à un échange potassium en mettant les granulés ci-dessus en contact avec une solution aqueuse de KCl suivant le même protocole expérimental que dans l'exemple 8.

On lui fait alors subir 2 opérations d'échange au baryum identiques à celles décrites à l'exemple 8.

Après toutes ces opérations, le solide est enfin activé à une température de 200°C pendant 2 heures sous courant d'azote.

Il présente les caractéristiques suivantes taux d'échange en baryum : 75,5% ; taux d'échange en potassium : 23%.

Le volume microporeux mesuré selon la méthode de Dubinin par adsorption d'azote à 77 K après prétraitement à 500°C pendant 12 heures sous vide est de 0,244 $cm^3$/g et sa perte au feu à 900°C est de 6,4%.

## Revendications

1. Adsorbant zéolitique aggloméré à base de cristaux de zéolite LSX de diamètre moyen en nombre compris entre 0,1 μm et 4 μm, de rapport atomique Si/Al = 1,00 ± 0,05, dont au moins 90% des sites cationiques échangeables sont occupés soit par des ions baryum seuls soit par des ions baryum et des ions potassium, **caractérisé en ce que** la résistance mécanique mesurée par la méthode Shell série SMS1471-74 adaptée aux agglomérés de taille inférieure à 1,6 mm est supérieure ou égale à 2 MPa.

2. Adsorbant zéolitique aggloméré selon la revendication 1, dont le volume de Dubinin est supérieur ou égal à 0,200 cm³/g, de préférence supérieur ou égal à 0,220 cm³/g, de préférence encore supérieur ou égal à 0,225 cm³/g.

3. Adsorbant zéolitique aggloméré selon la revendication 1, dont les sites échangeables occupés par le potassium peuvent représenter jusqu'à 1/3 des sites échangeables occupés par les ions baryum + potassium, et dont le complément éventuel est généralement assuré par des ions alcalins ou alcalino-terreux autres que le baryum et le potassium.

4. Adsorbant zéolitique aggloméré selon la revendication 1, comprenant un liant inerte en proportion inférieure ou égale à 20% en poids, de préférence 15% en poids, de la masse totale de l'aggloméré.

5. Adsorbant zéolitique aggloméré selon l'une quelconque des revendications précédentes, présentant une distribution de taille donnant un diamètre moyen compris entre 0,3 mm et 1,6 mm.

6. Adsorbant zéolitique aggloméré selon l'une quelconque des revendications précédentes, dont le taux global d'échange en baryum seul ou en baryum + potassium est supérieur ou égal à 95%.

7. Adsorbant zéolitique aggloméré selon l'une quelconque des revendications précédentes, dont la perte au feu mesurée à 900°C est comprise au sens large entre 4,0 et 7,7% et de préférence entre 4,7 et 6,7%.

8. Procédé de préparation d'un adsorbant zéolitique aggloméré selon l'une quelconque des revendications précédentes, comprenant les étapes suivantes :

    a) agglomération de cristaux de zéolite LSX, avec un liant d'agglomération, qui contient de préférence au moins 80% en poids d'argile(s) zéolitisable(s) (partie zéolitisable), et éventuellement une source de silice, puis mise en forme, séchage et calcination de la poudre agglomérée, le diamètre moyen en nombre des cristaux de zéolite LSX étant compris entre 0,1 μm et 4 μm, de préférence entre 0,1 μm et 3 μm, de préférence encore entre 0,1 μm et 2 μm,
    b) zéolitisation de ladite partie zéolitisable du liant par action d'une solution alcaline basique,
    c) remplacement d'au moins 90% des sites échangeables du produit obtenu à l'étape b) par du baryum, suivi du lavage et du séchage du produit ainsi traité,
    d) éventuellement remplacement d'au plus 33% des sites échangeables du produit obtenu à l'étape
    c) par du potassium, suivi du lavage et du séchage du produit ainsi traité, et e) activation éventuelle du produit obtenu, étant entendu que l'échange éventuel au potassium (étape d)) peut être pratiqué avant et/ou après l'échange au baryum (étape c)).

9. Procédé selon la revendication 8, dans lequel, dans l'étape a), le liant d'agglomération est en proportion inférieure ou égale à 20% en poids par rapport à l'aggloméré total.

10. Procédé selon l'une quelconque des revendications 8 ou 9, dans lequel l'activation de l'étape e) est une activation thermique exécutée à une température comprise entre 200°C et 300°C.

11. Procédé selon l'une quelconque des revendications 8 à 10, dans lequel la solution alcaline basique de l'étape b) a une concentration d'au moins 0,5 M.

12. Procédé de séparation de sucres, d'alcools polyhydriques, d'isomères de toluène substitué, des crésols ou de récupération de paraxylène, au moyen d'un adsorbant zéolitique aggloméré selon l'une quelconque des revendications 1 à 7, en présence d'un désorbant.

13. Procédé selon la revendication 12, de récupération de paraxylène à partir de coupes d'isomères C8 aromatiques, par adsorption du paraxylène au moyen d'un adsorbant zéolitique aggloméré selon l'une quelconque des revendications 1 à 7, en présence d'un désorbant.

14. Procédé selon l'une quelconque des revendications 12 ou 13, dans lequel le désorbant est choisi parmi le toluène et le paradiéthylbenzène.

**Patentansprüche**

1. Agglomeriertes zeolitisches Adsorptionsmittel auf der Grundalge von LSX-Zeolitkristallen mit einem mittleren Durchmesser im Bereich zwischen 0,1 $\mu$m und 4 $\mu$m, mit einem Atomverhältnis Si/Al = 1,00 $\pm$ 0,05, wovon mindestens 90 % der austauschbaren kationischen Stellen entweder durch Bariumionen allein oder durch Bariumionen und Kaliumionen besetzt sind, **dadurch gekennzeichnet, dass** der mechanische Widerstand, gemessen durch das Shell-Verfahren der Serie SMS1471-74, angepasst an die Agglomerate mit einer Größe von weniger als 1,6 mm, größer als oder gleich wie 2 MPa ist.

2. Agglomeriertes zeolitisches Adsorptionsmittel nach Anspruch 1, dessen Volumen von Dubinin größer als oder gleich wie 0,200 cm$^3$/g ist, vorzugsweise größer als oder gleich wie 0,220 cm$^3$/g, noch bevorzugter größer als oder gleich wie 0,225 cm$^3$/g.

3. Agglomeriertes zeolitisches Adsorptionsmittel nach Anspruch 1, wobei die austauschbaren Stellen, die von Kalium besetzt sind, bis zu 1/3 der austauschbaren Stellen darstellen können, die von den Barium- und Kaliumionen besetzt sind; und wobei der eventuelle Zusatz im Allgemeinen von Alkali- oder Erdalkaliionen außer Barium oder Kalium sichergestellt wird.

4. Agglomeriertes zeolitisches Adsorptionsmittel nach Anspruch 1, umfassend ein inertes Bindemittel in einer Proportion von weniger als oder gleich 20 Gew%, vorzugsweise 15 Gew% der gesamten agglomerierten Masse.

5. Agglomeriertes zeolitisches Adsorptionsmittel nach einem der vorhergehenden Ansprüche, das eine Größenverteilung aufweist, die einen durchschnittlichen Durchmesser im Bereich zwischen 0,3 mm und 1,6 mm ergibt.

6. Agglomeriertes zeolitisches Adsorptionsmittel nach einem der vorhergehenden Ansprüche, dessen globale Austauschquote mit Barium allein oder mit Barium und Kalium größer als oder gleich wie 95 % ist.

7. Agglomeriertes zeolitisches Adsorptionsmittel nach einem der vorhergehenden Ansprüche, dessen Glühverlust, gemessen bei 900 °C, im weiteren Sinne im Bereich zwischen 4,0 und 7,7 %, vorzugsweise zwischen 4,7 und 6,7 % liegt.

8. Verfahren zur Herstellung eines agglomerierten zeolitischen Adsorptionsmittels nach einem der vorhergehenden Ansprüche, umfassend die folgenden Schritte:

a) Agglomerieren von LSX-Zeolitkristallen mit einem Agglomerationsbindemittel, das vorzugsweise mindestens 80 Gew% zeolitisierbare(n) Ton/Töne (zeolitisierbarer Teil) enthält, und eventuell eine Siliciumquelle, dann Formen, Trocknen und Kalzinieren des agglomerierten Pulvers, wobei der mittlere Durchmesser der LSX-Zeolitkristalle im Bereich zwischen 0,1 $\mu$m und 4 $\mu$m liegt, vorzugsweise zwischen 0,1 $\mu$m und 3 $\mu$m, noch bevorzugter zwischen 0,1 $\mu$m und 2 $\mu$m,
b) Zeolitisieren des zeolitisierbaren Teils des Bindemittels durch die Wirkung einer basischen Lauge,
c) Ersetzen von mindestens 90 % der austauschbaren Stellen des Produkts, erhalten in Schritt b) durch Barium, gefolgt vom Waschen und Trocknen des so behandelten Produkts,
d) eventuell Ersetzen von höchstens 33 % der austauschbaren Stellen des Produkts, erhalten in Schritt c), durch Kalium, gefolgt vom Waschen und Trocknen des so behandelten Produkts, und
e) eventuelles Aktivieren des erhaltenen Produkts, wobei davon ausgegangen wird, dass der eventuelle Austausch mit Kalium (Schritt d)) vor und/oder nach dem Austausch des Bariums (Schritt c)) durchgeführt werden kann.

9. Verfahren nach Anspruch 8, wobei, in Schritt a) das Agglomerationsbindemittel in einer kleineren oder gleichen Proportion wie 20 Gew% mit Bezug auf das gesamte Agglomerat ist.

**10.** Verfahren nach einem der Ansprüche 8 oder 9, wobei die Aktivierung von Schritt e) eine thermische Aktivierung ist, durchgeführt bei eine Temperatur im Bereich zwischen 200 °C und 300 °C.

**11.** Verfahren nach einem der Ansprüche 8 bis 10, wobei die basische Alkalilösung von Schritt b) eine Konzentration von mindestens 0,5 M aufweist.

**12.** Verfahren zum Trennen von Zuckern, mehrwertigen Alkoholen, substituierten Toluenisomeren, Kresolen oder zur Wiedergewinnung von Paraxylen mit Hilfe eines agglomerierten zeolitischen Adsorptionsmittels nach einem der Ansprüche 1 bis 7 in Anwesenheit eines Desorptionsmittels.

**13.** Verfahren nach Anspruch 12 zur Widergewinnung von Paraxylen ausgehend von Schnitten von aromatischen C8-Isomeren durch Adsorption des Paraxylens mit Hilfe eines agglomerierten zeolitischen Adsorptionsmittels nach einem der Ansprüche 1 bis 7 in Anwesenheit eines Desorptionsmittels.

**14.** Verfahren nach einem der Ansprüche 12 oder 13, wobei das Desoprtionsmittel ausgewählt ist aus Toluen und Paradiethylbenzol.

## Claims

**1.** An aggregate zeolitic adsorbent based on LSX zeolite crystals having a mean number diameter of between 0.1 $\mu$m and 4 $\mu$m, a Si/Al atomic ratio=1.00$\pm$0.05, whereof at least 90% of the exchangeable cationic sites are occupied either by barium ions or by barium ions and potassium ions, **characterized in that** the mechanical strength measured by the Shell series SMS1417-74 method adapted to the aggregates having a size smaller than 1.6 mm is greater than or equal to 2 MPa.

**2.** The aggregate zeolitic adsorbent according to claim 1, whereof the Dubinin volume is greater than or equal to 0.200 cm$^3$/g, preferably greater than or equal to 0.220 cm$^3$/g, even more preferably greater than or equal to 0.225 cm$^3$/g.

**3.** The aggregate zeolitic adsorbent according to claim 1, whereof the exchangeable sites occupied by potassium may account for up to 1/3 of the exchangeable sites occupied by the barium plus potassium ions, and whereof the optional complement is generally provided by alkali or alkaline-earth ions other than barium and potassium.

**4.** The aggregate zeolitic adsorbent according to claim 1, comprising an inert binder in a proportion of less than or equal to 20% by weight, preferably 15% by weight, of the total mass of aggregate.

**5.** The aggregate zeolitic adsorbent according to any one of the preceding claims, having a size distribution giving a mean diameter of between 0.3 mm and 1.6 mm.

**6.** The aggregate zeolitic adsorbent according to any one of the preceding claims, whereof the total exchange rate of barium alone or of barium plus potassium is greater than or equal to 95%.

**7.** The aggregate zeolitic adsorbent according to any one of the preceding claims, whereof the ignition loss measured at 900°C is broadly between 4.0 and 7.7% and preferably between 4.7 and 6.7%.

**8.** A method for preparing an aggregate zeolitic adsorbent according to any one of the preceding claims, comprising the following steps:

a) agglomeration of LSX zeolite crystals, with an agglomeration binder, which preferably contains at least 80% by weight of zeolitizable clay(s) (zeolitizable portion), and optionally a source of silica, followed by shaping, drying and calcination of the agglomerated powder, the number average diameter of the LSX zeolite crystals being comprised between 0.1 $\mu$m and 4 $\mu$m, preferably between 0.1 $\mu$m and 3 $\mu$m, more preferably between 0.1 $\mu$m and 2 $\mu$m,
b) zeolitization of said zeolitizable portion of the binder by the action of a basic alkaline solution,
c) replacement of at least 90% of the exchangeable sites of the product obtained in step b) by barium, followed by washing and drying of the product thus treated,
d) optionally, replacement of no more than 33% of the exchangeable sites of the product obtained in step c) by potassium, followed by washing and drying of the product thus treated, and

e) optional activation of the product obtained, it being understood that the optional exchange with potassium (step d)) may be carried out before and/or after the exchange with barium (step c)).

9. The method as claimed in claim 8, wherein, in step a), the agglomeration binder is in a proportion lower than or equal to 20% by weight of the total aggregate.

10. The method according to any one of claims 8 or 9, wherein the activation in step e) is a thermal activation, carried out at a temperature between 200°C and 300°C.

11. The method according to any one of claims 8 to 10, in which the basic alkaline solution in step b) has a concentration of at least 0.5 M.

12. A method for separating sugars, polyhydric alcohols, isomers of substituted toluene, cresols, or for recovering paraxylene, using an aggregate zeolitic adsorbent according to any one of claims 1 to 7, in the presence of a desorbent.

13. The method according to claim 12, for recovering paraxylene from aromatic C8 isomer cuts, by adsorption of the paraxylene, using an aggregate zeolitic adsorbent according to any one claims 1 to 7, in the presence of a desorbent.

14. The method according to any one of claims 12 or 13, wherein the desorbent is selected from toluene and paradiethylbenzene.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 3558730 A
- US 3558732 A
- US 3626020 A
- US 3663638 A
- US 3960774 A
- US 3878127 A
- US 2985589 A
- US 2882244 A
- US 3130007 A
- US 6884918 B
- US 6410815 B

- US 3119660 A
- US 4818508 A
- JP 3066430 B
- EP 1105213 A
- US 5284992 A
- US 5629467 A
- US 4402832 A
- US 4498991 A
- EP 0486384 A
- US 5173462 A

**Littérature non-brevet citée dans la description**

- **D.W. BRECK.** Zeolite Molecular Sieves. John Wiley and Sons, 320
- **RUTHVEN.** *Principles of Adsorption and Adsorption Processes» («Principes de l'Adsorption et des procédés d'adsorption»,* 326-407
- **RUTHVEN.** Principles of Adsorption and Adsorption processes. 248-250

- **LI.** Primary analysis on state of xylene adsorption unit. *Jingxi Shiyou Huagong,* 2004, vol. 4, 54-55
- Characterization of Porous Solids and Powders : Surface Area, Pore Size and Density. **LOWELL.** Micropore Analysis. 143-145